Europäisches Patentamt

European Patent Office

Office européen des brevets

(11) Veröffentlichungsnummer: **0 222 974 B1**

# EUROPÄISCHE PATENTSCHRIFT

(45) Veröffentlichungstag der Patentschrift: **13.11.91**

(51) Int. Cl.⁵: **A61K 39/205**

(21) Anmeldenummer: **86110116.0**

(22) Anmeldetag: **23.07.86**

Die Akte enthält technische Angaben, die nach dem Eingang der Anmeldung eingereicht wurden und die nicht in dieser Patentschrift enthalten sind.

(54) Verfahren zur Herstellung eines Tollwutimpfstoffes und nach diesem Verfahren erhaltener Impfstoff.

(30) Priorität: **22.11.85 CH 4999/85**

(43) Veröffentlichungstag der Anmeldung:
**27.05.87 Patentblatt 87/22**

(45) Bekanntmachung des Hinweises auf die Patenterteilung:
**13.11.91 Patentblatt 91/46**

(84) Benannte Vertragsstaaten:
**AT BE CH DE FR GB IT LI NL SE**

(56) Entgegenhaltungen:
**DE-B- 1 176 317**
**FR-A- 2 453 651**

(73) Patentinhaber: **Schweizerisches Serum- und Impfinstitut und Institut zur Erforschung der Infektionskrankheiten**
**Rehhagstrasse 79**
**CH-3018 Bern(CH)**

(72) Erfinder: **Glück, Reinhard, Dr. phil. nat.**
**Blinzernfeldweg 8**
**CH-3098 Köniz(CH)**
Erfinder: **Germanier, René, prof. Dr. phil. nat.**
**Mettlenhölzliweg 8**
**CH-3074 Muri bei Bern(CH)**

(74) Vertreter: **Zutter, Hans Johann Niklaus**
**EPROVA AG Forschungsinstitut Im Laternenacker 5**
**CH-8200 Schaffhausen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues oekonomisches Verfahren zur Herstellung eines Tollwutimpfstoffes, bestehend aus vollständigen intakten Viren, die durch chemische Behandlung vermehrungsunfähig gemacht werden sowie den nach diesem Verfahren erhaltenen Impfstoff.

Der nach diesem Verfahren erhaltene Impfstoff zeichnet sich aufgrund seiner hohen Reinheit durch eine hohe spezifische Wirksamkeit und das Fehlen von unerwünschten Impfnebenreaktionen aus.

Der überwiegende Teil der Tollwutimpfstoffe wird heute noch durch Vermehrung der Viren in lebenden Tieren wie Mäusen, Ratten, Kaninchen, Schafen etc. gewonnen, obwohl dabei virushaltiges Gewebe anfällt, das erhebliche Mengen Myelin enthält.

In neuerer Zeit wurden Tollwutimpfstoffe auch aus Viren gewonnen, die in Geflügelembryonen vermehrt wurden. Dies hat den prinzipiellen Vorteil, dass das dabei entstehende virushaltige Gewebe kaum schädliches Myelin enthält. Nach der Vermehrung der Viren in Geflügelembryonen wurden diese Embryonen in toto in einem Mixer oder Blender vollständig homogenisiert. Aus dem breiigen Homogenisat lassen sich die Viren-bestandteile nur unvollkommen heterologem Protein abtrennen, das beim Impfen unerwünschte Nebenreaktionen auslösen kann. Dasselbe gilt für Impfstoffe, die aus den mit Tollwut infizierten Gehirnen lebender Tiere gewonnen wurden. Diese Nebenreaktionen können sich bei widerholtem Impfen, das bei gewissen Berufsleuten wie Jägern, Förstern, Tierärzten unerlässlich ist, zu heftigen allergischen Abwehrreaktionen gegen das artfremde Eiweiss steigern.

Eine Verbesserung der Impfstoffqualität liess sich dadurch erzielen, dass man zur Impfstoffgewinnung nur die Köpfe der Embryonen verwendet hat, welche eine vergleichsweise wesentlich höhere Konzentration an Viren aufweisen. Der nur aus Embryonenköpfen hergestellte Impfstoff weist einen entsprechend geringeren Gehalt an Nebenprodukten auf und verursacht weniger Nebenwirkungen. Siehe dazu: Deutsche Patentschrift DE 30 09 064, amerikanische Patentschrift 4,255,520. Bei der Impfstoffherstellung aus Nervengewebe von Tieren, aus Embryonen oder aus Embryonenköpfen werden beim Homogenisieren des virushaltigen Gewebes mit einem Mixer oder Blender jedoch gesplittete Virusbestandteile extrahiert. Dies vermindert die Wirksamkeit des aus solchen Homogenisaten hergestellten Impfstoffes erheblich und erschwert dessen Reinigung enorm, da grosse Mengen von Proteinen und Lipiden aus den zerstückelten Zellen freigesetzt werden.

Ein besserer Impfstoff liess sich bisher nur durch Vermehrung der Tollwutviren in menschlichen Diploidzell (HDC)-Kulturen in vitro erhalten.

Vergleiche dazu: H. Koprowski, Laboratory Techniques in Rabies by M.M. Kaplan und H. Koprowski, WHO Monograph Series No. 23, Chapter 28, pp. 256-60 (1973): Vaccine for man prepared in human diploid cells; T.J. Wiktor, Develop. biol. Standard, Vol. 37, pp. 256-66, S. Karger, Basel 1978: Production and control of rabies vaccines made on diploid cells;

T.J. Wiktor et al, Develop. biol. Standard, Vol. 40, pp. 3-9 (1978): Development and clinical trial of rabies vaccine of tissue cultur. Der so gewonnene Impfstoff enthält als Verunreinigung humane Proteine, welche weniger Nebenreaktionen erzeugen als artfremde Proteine.

Ein wesentlicher Nachteil dieser Methode ist die relativ geringe Vermehrungsrate der Tollwutviren auf diploiden Fibroblaszellen, was eine 10- bis 25fache Aufkonzentrierung für die Impfstoffherstellung nötig macht. Diese Methode ist daher nicht leistungsfähig genug, um den weltweiten Bedarf an Tollwutimpfstoff im Rahmen der wirtschaftlichen Möglichkeiten zu decken.

In der US-PS 3,674,862 wird die Herstellung eines Tollwutimpfstoffes in Entenembryo-Zellkulturen beschrieben. Die Vermehrungsrate in Zellkulturen ist limitiert. Die US-PS 3,973,000 beschreibt eine Methode zur Anreicherung von Tollwutviren durch Dichtegradientenzentrifugation. M. Rolle und A. Mayr: Mikrobiologie, Infektions-und Seuchenlehre, Stuttgart 1978, S. 489-493 beschreiben die traditionelle Herstellung von Entenembryo-Tollwutimpfstoff.

Es besteht daher ein dringender Bedarf an einem neuen hochwirksamen Tollwutimpfstoff, welcher mechanisch intakte Viren mit voll erhaltener antigener Wirksamkeit enthält, der einfach herzustellen und damit nicht zu teuer ist und der keine Nebenwirkungen verursacht. Ein solcher Impfstoff wäre das seit langem gesuchte wirksame und gut verträgliche Mittel zur Bekämpfung der praktisch weltweit auftretenden schrecklichen Krankheit, die sich in fataler Weise ausbreitet.

Ziel der vorliegenden Erfindung ist es demnach, ein ökonomisches Verfahren zu entwickeln, das einen Tollwutimpfstoff höchster Qualität liefert, der sich mit dem Impfstoff vergleichen lässt, welcher aus Viren gewonnen wurde, die in diploiden Humanzellkulturen vermehrt wurden.

Es wurde gefunden, dass sich dieses hochgesteckte Ziel durch Anwendung folgender Verfahrensschritte weitgehend erreichen lässt:

1. Die in tierischem Nervengewebe, vorzugsweise in Geflügelembryonen vermehrten Tollwutviren werden

EP 0 222 974 B1

unter Vermeidung von Kontaminationen mit intrazellulären Splitprodukten isoliert.

Das Extrahieren des Tollwutvirus erfolgt dabei durch vorsichtige zellschonende Zerkleinerung des Nervengewebes bzw. der sterilen Entenembryonenköpfe und durch Spülen der Gewebefragmente mit einem phosphat-haltigen Puffer. Dieses Verfahren ist vorteilhafter als das Homogenisieren in einem Mixer oder Blender. Fremdproteine und Lipide werden in geringerem Masse solubilisiert, das Auftreten von Oxydationsprodukten von Antigenen, Proteinen und Lipiden wird vermieden und der Gehalt an intrazellulärem, unvollständigem und nichtimmunisierendem Tollwutantigen wird verringert. Die beim Spülen der Gewebsfragmente mit einer wässrigen Pufferlösung erhaltene virus-haltige Suspension wird durch Differentialzentrifugation abgetrennt.

2. Aus dem erhaltenen Viruskonzentrat werden durch Extraktion mit einem mit Wasser nicht mischbaren organischen Lösungsmittel, wie z.B. mit flüchtigen Paraffinkohlenwasserstoffen oder fluorierten Kohlenwasserstoffen, die Fremd-Lipide entfernt.

3. Das delipidierte Virus-konzentrat wird durch Dichtegradientenzentrifugation weiter angereichert und/oder

4. die Viren werden durch Zusatz von Polyethylenglykol (z.B. PEG 6000) ausgefällt und durch Zentrifugation konzentriert und gereinigt.

Durch die Operationen 3 und/oder 4 werden mindestens 95 % des Restproteins abgeschieden.

Schliesslich werden die Viren auf bekannte Weise inaktiviert, beispielsweise durch Zusatz von Beta-Propiolacton oder Tri-(n-butyl)phosphat.

Die einzelnen Verfahrensschritte sind derart aufeinander abgestimmt, dass ein überraschend gutes Gesamtergebnis erzielt wird.

Durch Ernten der Viren nur aus den Köpfen der Embryonen wird eine hohe Basiskonzentration der Viren erhalten. Die schonende Behandlung des virushaltigen Gewebematerials, insbesondere durch das Vermeiden von dessen Homogenisierung mit einem Mixer zu einem feinen Brei, liefert eine Virussuspension, die kaum zerstückelte Viren mit unvollständigem Antigengehalt aufweisen und die zudem weit weniger Fremdstoffe wie Zelltrümmer, Proteine und Lipide enthält. Aus dieser Virussuspension lassen sich die restlichen Lipide durch Extraktion und die Proteine durch selektive Konzentration und/oder Ausfällung der Viren weit vollständiger entfernen als aus einem breiigen Homogenisat.

Der nach der beschriebenen Verfahrensfolge gewonnene Impfstoff ist gegenüber dem herkömmlichen Entenembryo-Tollwutimpfstoff um das 90fache verbessert.

Das vorliegende neue Verfahren lässt sich aber auch ausdehnen auf die Herstellung von Tollwutimpfstoffen aus dem Nervengewebe von Tieren wie Mäusen, Ratten, Kaninchen und Schafe etc., in denen durch intracerebrale Inokulation von Tollwutviren aus standardisierten Saatstämmen die Viren im lebenden Tier vermehrt wurden.

Die Vermehrung der Viren in lebenden Tieren hat allerdings gegenüber der Virusvermehrung in Geflügelembryonen den Nachteil, dass das Nervengewebe der lebenden Tiere Myelin enthält, das bei der Anwendung des Impfstoffes Anlass zu Nebenreaktionen gibt, die sich bis zur Encephalitis steigern können.

Den Tieren werden nach der Schlachtung die Gehirne entnommen, zellschonend zerkleinert und aus der erhaltenen Zellsuspension der Impfstoff nach der oben beschriebenen erfindungsgemässen Verfahrensfolge hergestellt.

Durch die Vermeidung der Zellzertrümmerung bei der Zerkleinerung des virushaltigen Nervengewebes wird weniger Myelin freigesetzt als bei der Zerkleinerung mit einem Mixer. Bei der Extraktion der Lipide mit einem organischen Lösungsmittel in einer späteren Phase der Impfstoffherstellung wird ein weiterer Teil des noch vorhandenen Myelins entfernt, derart, dass schliesslich ein Impfstoff erhalten wird, der nur noch geringe tolerierbare Nebenreaktionen verursacht.

Gegenstand der Erfindung ist demnach das Verfahren zur Herstellung eines Tollwutimpfstoffes durch Vermehrung von Tollwutviren in tierischem Nervengewebe, vorzugsweise in Geflügelembryonen, Ernte der Viren aus dem Nervengewebe bzw. aus den Köpfen der Embryonen, Anreicherung der Viren, Inaktivierung derselben und Konfektionierung zum Impfstoff, dadurch gekennzeichnet, dass man bei der Ernte der Viren die Homogenisierung des Nervengewebes bzw. der Embryonenköpfe mit einem Mixer oder Blender vermeidet und dadurch die Isolierung unvollständiger Viren verhindert, indem man das Nervengewebe der Tiere bzw. die Köpfe der Embryonen bzw. deren Inhalt zellschonend zerkleinert, aus dabei erhaltener Zellsuspension die intakten, lebenden und vermehrungsfähigen Viren herausspült, die Virussuspension gereinigt, durch Extraktion mit einem mit Wasser nicht mischbarem organischen Lösungsmittel von Lipiden befreit und danach selektiv konzentriert.

Die Zerkleinerung des Nervengewebes bzw. der Embryonenköpfe bzw. von deren Inhalt erfolgt mit Hilfe eines grob eingestellten Fleischwolfes, durch Zerschneidung oder durch Oeffnen der Köpfe und zellschonendes Zerkleinern des entnommenen Gehirngewebes.

3

Das Herausspülen bzw. die Extraktion der Viren aus dem zerkleinerten Gewebe erfolgt mit einer Pufferlösung, vorzugsweise mit einem wässrigen Phosphatpuffer vom pH 7 - 8.

Die Entfernung der Lipide erfolgt durch Extraktion mit flüssigen, leicht flüchtigen gegebenenfalls halogenierten Kohlenwasserstoffen, beispielsweise mit Petrolether wie Heptanen, mit fluorierten und chlorierten Ethanen oder mit nahen Homologen derselben.

Die weitere Konzentrierung der delipidierten Virussuspension erfolgt durch Dichtegradientenzentrifugation und/oder Fällung mit einem Polyethylenglykol, vorzugsweise mit PEG 6000.

Geeignete Geflügelarten für die Vermehrung von Tollwutviren in deren Embryonen sind insbesondere Enten, Hühner und Wachteln.

Im allgemeinen werden bebrütete Enteneier als Gewebebasis für die Vermehrung der Viren vorgezogen.

Gegenstand der Erfindung sind auch myelinfreier Tollwutimpfstoff, der dadurch gekennzeichnet ist, dass er aus Tollwutviren enthaltenden Geflügelembryonenkopfgewebe nach dem vorstehend beschriebenen zellschonenden Verfahren gewonnen wurde.

Durch das erfindungsgemässe Verfahren wird ein Tollwutimpfstoff erhalten, der, verglichen mit nach bisher bekannten Verfahren gewonnenen Impfstoffen, ein weit besseres Verhältnis von Antigengehalt zu Proteingehalt aufweist, keine Fremd-Lipide enthält und qualitativ einem HDC-Idealimpfstoff nahekommt.

Detaillierte Beschreibung der Verfahrens

1. Ein für die Impfstoffherstellung geeigneter Virusstamm wird durch entsprechende Passagen an die Embryonenzellen in Geflügeleiern bzw. in Mäusen, Ratten, Kaninchen oder Schafen u.a. dem vorgesehenen Viruswirt angepasst.

Attenuierte Tollwutviren werden z.B. in den Dottersack von befruchteten und angebrüteten Geflügeleiern inokuliert, in denen sich ein Embryo entwickelt. Nach etwa 2 Wochen werden die Embryonen entnommen und deren Köpfe geerntet.

Die Embryonenköpfe werden in einem Fleischwolf zellschonend zerkleinert oder das Gehirngewebe wird nach Zerschneiden oder Oeffnen des Kopfes entnommen und zerkleinert.

Wenn die Vermehrung der Viren in lebenden Tieren stattfindet, wird das artspezifisch attenuierte Saatvirus gewöhnlich nur wenige Tage alten Tieren (Mäusen, Ratten, Kaninchen, Lämmern etc.) intracerebral inokuliert. Nach etwa 10 - 30 Tagen werden die Tiere getötet, die Gehirne werden herausoperiert und zellschonend zerkleinert.

Das Extrahieren des Tollwutvirus aus dem zerkleinerten Gewebe erfolgt durch Spülen der Gewebefragmente mit einem phosphat-haltigen Puffer. Neben Phosphat-Puffer vom pH 7,4, enthaltend z.B. 0,75 Gewichtsprozent Dinatriumhydrogenphosphat, 0,145 Gewichtsprozent Kaliumdihydrogenphosphat und 0,48 Gewichtsprozent Natriumchlorid in destilliertem Wasser, ist es ebenso gut möglich Stabilisierungsmittel und Salzlösungen, wie sie für die Herstellung von Virusvakzinen-suspensionen allgemein üblich sind, oder sogar entsalztes Wasser für die Extraktion zu verwenden, sofern der pH-Wert im Bereich zwischen 7 und 8 liegt.

Die virusantigen-haltige Suspension wird durch Differentialzentrifugation bei ca. 10.000 - 15.000 xg (xg = x mal die Gravitationsbeschleunigung) von dem Gewebe abgetrennt. Das verbleibende Gewebesediment kann für eine weitere Extrahierung verwendet werden, wodurch ein Virusantigen-Gewinn von ca. 30 % ermöglicht wird. Beide virushaltigen Extrakte werden vereinigt und filtriert.

2. Durch Extraktion mit einem mit Wasser nicht mischbaren organischen Lösungsmittel wie etwa mit einem gegebenenfalls fluorierten Kohlenwasserstoff werden die noch in der Virussuspension verbliebenen Fremdlipide entfernt.

Anschliessend wird das Antigenextrakt (= Virussuspension) durch Dichtegradientenzentrifugation in an sich bekannter Weise bei 15.000 - 90.000 xg mittels Puffer- und Zuckerlösungen verschiedener Konzentration, d.h. in steigenden Zuckerkonzentrationen angereichert und/oder durch Ausfällung mit Polyethylenglykol konzentriert.

3. Die Dichtegradientenzentrifugation wird in an sich bekannter Weise bei 15 000 bis 90 000 xg mit Hilfe verschieden konzentrierter Zuckerlösungen und von Pufferlösungen, d.h. in steigenden Zuckerkonzentrationen und in Pufferlösung, in an und für sich bekannter Weise durchgeführt. Dazu wird die vorgereinigte Suspension bei 15 000 bis 90 000 xg und einer Durchflussrate von 4 Lit./h über eine vorgelegte Stufengradiente zunehmender Konzentrationen von Zucker (gewöhnlich Saccharose von 15 bis 55 %) gepumpt. An den Fraktionen, die von den verschiedenen Dichten gesammelt werden, werden Dichte, Lipoid-, Nukleoprotein- und Antigen-Gehalt sowie Sterilität bestimmt. Die antigenhaltigen Fraktionen werden gepoolt, nochmals geprüft und dann zur Vakzine weiterverarbeitet. Physiologische Salzlösungen jeder Art, z.B. der weiter oben genannten Phosphatpuffer, können zur Verdünnung eingesetzt werden. In diesem Zusammenhang wird auf folgende Publikationen verwiesen: Entenembryo Tollwutvakzine: J.M. Hoskins, Laboratory Techniques in Rabies by M.M. Kaplan et al., WHO Genf 1973, Kapitel 27, S. 243 - 55. Dichtegradientenzentrifugation: J. Hilfenhaus et al., Journal of Biological Standardization 1976, 4, 263 - 271; M. Majer et al., Develop. biol. Standard., Band 37, S. 267 - 271 (1977) und P. Atanasiu et al., Develop. biol. Standard., Band 40, S. 35 - 44.

4. Zusätzlich oder alternativ zu der Anreicherung der Viruskonzentration durch Dichtegradientenzentrifugation wird die vorgereinigte und gewöhnlich angereicherte Virussuspension durch Ausfällung mit einem Polyethylenglykol weiter konzentriert und gereinigt, d.h. vor allem von heterologem Protein befreit.

Dazu wird das pH der Virussuspension auf 8 eingestellt. Nach Zugabe von Polyethylenglykol (z.B. PEG 6000) bis zu einer Endkonzentration von 6 Gewichtsprozent wird die Suspension mindestens eine Stunde lang gerührt. Durch anschliessendes Zentrifugieren bei 10.000 - 15.000 xg wird das Virus ausgefällt. Das Virussediment wird in einem Stabilisierungsmittel bestehend aus einer Lösung enthaltend Lactose und physiologische Gelatine erneut suspendiert. Siehe in diesem Zusammenhang: E.M. Mikhailovsky et al., Ann. Inst. Pasteur, 1971, Band 121, S. 563 - 568; James McSlarry et al., Virology, 1970, Band 40, S. 745 - 746.

5. Die im erhaltenen Viruskonzentrat enthaltenen intakten lebenden und vermehrungsfähigen Viren werden nun inaktiviert. Gewöhnlich wird für die Inaktivierung Beta-Propiolacton (BPL) verwendet. Vergleiche in diesem Zusammenahng: G.A. LoGrippo, Annals New York Academy of Sciences, Band 83 (1960), S. 578 - 94. Es sind jedoch auch andere Substanzen dazu geeignet, beispielsweise Tri-(n-butyl)phosphat. H. Tint et al., Symposia series in immunobiological standardization (Karger, Basel), 21, 132 - 144: A new tissue culture rabies vaccine, inactivated and disaggregated with tri-(n-butyl) phosphate. T. J. Wiktor et al., Develop. biol. Standard., Band 40, S. 3 -9 (1978).

Das durch das neue Verfahren gewonnene Impfstoff-Konzentrat unterscheidet sich durch seinen hohen Gehalt von weit mehr als 100 Antigen-Werteinheiten (gemessen mit Hilfe des Standard NIH Tests bei Mäusen und des RFFIT Tests) pro mg Stickstoff von handelsüblichen Tollwutimpfstoffen. Es wurde in der Regel unter Verwendung von ungeborenen Embryonen gewonnen, die noch keinen Schmerz empfinden und bei denen das Gehirngewebe, das sich gerade erst entwickelt, noch frei von Myelin zu sein scheint. Vergleiche in diesem Zusammenhang: M. Abdussalen et al., The problem of anti-rabies vaccination, International conference on the application of vaccine against assay viral rickettsial and bacterial diseases of man, Pan. Am. Health Org. (PAHO), Sc. pub. Nr. 226 (1970), S. 54 - 59, und P. Fenje, The status of existing rabies vaccines, ibid. S. 60 -65.

6. Nach der Inaktivierung wird der erhaltene Impfstoff gewöhnlich in Fläschchen abgefüllt und lyophilisiert.

Für den Gebrauch wird er durch Auflösen bzw. Suspendieren mit destilliertem Wasser rekonstituiert.

Durch das beschriebene neue Verfahren können unbegrenzte oder doch wenigstens ausreichende Mengen des gewünschten wertvollen und unschädlichen Impfstoffs wirtschaftlich und relativ einfach hergestellt werden. Im Falle der Herstellung von Tollwutimpfstoff durch Vermehrung der Viren in menschlichen Diploidzell (HDC)-Kulturen ist dies infolge der geringen Effizienz dieses Substrates nicht möglich. Vergleiche: Anweisung des "Centers for Disease Control" (Zentrale zur Bekämpfung von Krankheiten), CDC, vom Februar 1979: Die CDC hat die Anwendung von Human-Diploidzell-Tollwutvakzinen auf Personen beschränkt, bei denen nach Gabe von Entenembryo-Vakzine lebensgefährdende Nebenwirkungen auftraten oder die ausserstande waren, einen angemessenen Titer von Antikörpern zu erlangen. Als Grund dafür wird die unzureichende Produktivität der menschlichen Diploidzell-Kulturen angegeben. Siehe auch: Morbidity and Mortality Weekly Report (MMWR) 27, 333, 413 (1978).

Wie am Schluss des nachfolgenden Beispiels 1 nachgewiesen wird, ist der nach dem erfindungsgemässen Verfahren hergestellte Tollwutimpfstoff einem HDC-Impfstoff, bei dem die Viren in menschlichen Diploidzell-Kulturen vermehrt wurden, mindestens gleichwertig. Nebenwirkungen wurden nicht beobachtet. Damit ist offensichtlich das eingangs gesteckte Ziel der vorliegenden Erfindung erreicht worden, der Medizin einen preiswerten und trotzdem ideal wirksamen Tollwutimpfstoff, der ein Minimum von Nebenwirkungen aufweist, zur Verfügung zu stellen.

Beispiel 1
Herstellung eines Entenembryo-Tollwutimpfstoffes.

1. Herstellung der Virussuspension

(a) Der "Wistar Tollwut, PM (Pitman-Moore)-8HDCS" Virus Stamm aus dem Wistar Institut, Philadelphia oder ein anderer zur Impfstoffherstellung geeigneter Tollwutvirusstamm wird vor der eigentlichen Verwendung durch intrazerebrale Passage in Mäusen und wiederholte Passagenimpfung in angebrüteten Enteneiern an die Embryonenzellen angepasst. Für die Vakzinezubereitung werden Viren aus einer Passage mit besonders hohem Titer verwendet, die sich in der Herstellung von Tollwutimpfstoff nach der Methode von J.M. Hoskins (Laboratory Techniques in Rabies by Kaplan et al., WHO, 1973, 27. S. 243 - 55: Duck Embryo Vaccine) bereits als geeignet erwiesen haben.

Befruchtete Enteneier aus gesunden Beständen werden bei einer Temperatur von 36° C ± 1° C und einer Feuchtigkeit von 65 - 70 % inkubiert. Nach sechs Tagen werden sie durchleuchtet, und ungeeignete Eier werden aussortiert. Am 7. Inkubationstag wird das Tollwutvirus direkt in den Dottersack der Eier inokuliert, in denen sich ein Embryo entwickelt. Die Inkubation wird fortgesetzt. 10 - 14 Tage später werden die Eier erneut mit UV-Licht durchleuchtet. Die Eier, in denen sich die Embryos weiter gut entwickelt haben, werden unter sterilen Bedingungen geöffnet; die Embryonen werden entnommen und geköpft. Die Köpfe werden einzeln unter sterilen Bedingungen in der Dampfphase über flüssigem Stickstoff aufbewahrt, bis die Sterilitätstests abgeschlossen sind. Jeweils 40 - 60 der sterilen Köpfe werden unter Zusatz einer bestimmten Menge eines Stabilisierungsmittels zu einem Pool vereinigt. Die Sterilität eines jeden Pools wird erneut geprüft. Neben dem Stabilisierungsmittel können auch NaCl/Phosphat-Puffer (= 0,75 % Dinatriumhydrogenphosphat, 0,145 % Kaliumdihydrogenphosphat und 0,48 % Natriumchlorid in destilliertem Wasser) oder andere Salzlösungen, wie sie in der Herstellung von Vakzinen für Verdünnungszwecke üblich sind, bis hin zu entsalztem Wasser verwendet werden, sofern der pH- Wert in dem Bereich zwischen 7 und 8 liegt.

Der Tollwutvirus-Extrakt wird durch Zerkleinern der oben erwähnten sterilen Embryonenköpfe mittels eines Fleischwolfs gewonnen. Die Gewebefragmente werden zweimal mit einem phosphat-haltigen Puffer gespült. Das infektiöse Virus wird nach Zentrifugieren bei 10.000 - 15.000 xg und einer Temperatur von 2 - 8 ° C in der überstehenden Fraktion aufgefangen. Durch anschliessendes Filtrieren über ein Gaze-Filter-System werden verbleibende Gehirnpartikel oder andere lipid-haltige Gewebe abgetrennt. Die verbleibenden Kopfgeweberückstände können mit Hilfe desselben Verfahrens noch einmal extrahiert und filtriert werden, wodurch ein höherer Antigenertrag von etwa 30 % erzielt wird. Das Sediment wird erneut mit einem phosphathaltigen Puffer aufgeschwemmt und vor dem Zentrifugieren und Filtrieren mindestens eine Stunde lang bei einer niedrigen Temperatur (1 - 4° C) gerührt.

(b) Die weitere praktisch vollständige Delipidierung erfolgt durch das Mischen der erhaltenen Virussuspension mit einem gleichen Volumen eines inerten, flüssigen Kohlenwasserstoff-Lösungsmittels mit einer relativ geringen Dichte, wie z.B. n-Heptan. Homogenisiert wird in jedem Fall unter einer Glasglocke mit Stickstoffgas. Bei einer gleichbleibenden Fliessgeschwindigkeit (z.B. 500 ml/min) wird die Virussuspension durch ein Mischersystem (z.B. Virtis-Mischer) gepumpt. Gleichzeitig wird das n-Heptan in einer Geschwindigkeit von 50 ml/min in das Mischersystem gepumpt. Die lipid-haltige Phase wird durch Zentrifugieren bei 10.000 - 15.000 xg abgetrennt. Dann wird der delipidierte Virusextrakt von Spuren des gelösten Kohlenwasserstoff-Lösungsmittels befreit und zwar dadurch, dass man ein inertes Gas wie z.B. Stickstoff durch die wässrige Phase hindurchperlen lässt und die wässrige Phase für einen Zeitraum von ca. 15 Stunden bei 4° C unter einem Vakuum hält.

(C) Ein alternatives Verfahren zur Delipidierung mit Kohlenwasserstoff ist folgendes: Sterile Embryonenköpfe werden, wie unter (1a) beschrieben, zerkleinert, extrahiert und filtriert. Die Abtrennung der Fremdlipide erfolgt unter Verwendung des fluorierten Kohlenwasserstoff-Lösungsmittels 1,1,2-Trichlortrifluorethan. Die einzelnen Arbeitsschritte bleiben dieselben.

2. Konzentrierung und weitere Reinigung der Virussuspension

(a) Die vorgereinigte, nach dem oben beschriebenen Verfahren bereitete Tollwutvirus-Suspension weist einen Virustiter zwischen $10^7$ und $10^8$ $MLD_{50}$/ml auf. Dieses Material wird weiter gereinigt und konzentriert, und zwar durch zweimaliges Zentrifugieren über einen linearen Saccharose-Gradienten (15 - 55 %) bei 75.000 - 90.000 xg. Auf diese Weise wird ein Konzentrationsfaktor von 100 : 1 erreicht. Glykoprotein- und Nukleoproteingehalt (vor und nach Solubilisierung der Virusmembran durch Triton X 100, d.h. Selektion der intakten Virionen). Virustiter, Dichte und Sterilität der Gradientenfraktionen werden geprüft. Sterile Fraktionen mit einem Verhältnis von Tollwut-Glykoprotein zu Nukleoprotein, das dem der gereinigten Ganzvirion-Lösung entspricht, und einem sehr hohen infektiösen Titer (z.B. $10^9$ -$10^{10}$ $MLD_{50}$/ml) werden vereinigt und für die Weiterverarbeitung vorgemerkt.

(b) Eine weitere Reinigung und Konzentrierung der Virussuspension kann mittels Polyethylenglykol (PEG)-Ausfällung erreicht werden. Dazu wird PEG 6000 (Siegfried A.G., Zofingen, Schweiz) in einer 30 %igen phosphathaltigen Pufferlösung (pH 8,0) gelöst. Diese PEG-Stammlösung wird im Autoklaven sterilisiert und kann bei 4° C aufbewahrt werden. Die Virussuspension, die mit einer 10 %igen NaOH-

6

Lösung auf einen pH-Wert von 8,0 eingestellt wurde, wird mit der PEG-Stammlösung bei einer Endverdünnung von 6 % ausgefällt. Das Gemisch wird bei einer Temperatur von 4 °C mindestens eine Stunde lang gerührt. Dann kann sich das Tollwutvirus beim Zentrifugieren mit einer Geschwindigkeit von 10.000 - 15.000 xg über einen Zeitraum von 30 min absetzen. Das abgetrennte Virus wird erneut mit einem Stabilisierungsmittel zu dem endgültigen Volumen aufgeschwemmt und für die weitere Verarbeitung vorgemerkt.

### 3. Formulierung der Viruskonzentrate

Vorgetestete Viruskonzentrate werden vereinigt und mit einem geeigneten Stabilisierungsmittel, z.B. Natriumphosphat-Puffer (pH 7,4), einer physiologischen Kochsalzlösung, oder mit einem anderen bereits beschriebenen Stabilisierungsmittel (siehe Hoskins, 1.c.) auf eine Konzentration von ca. $10^{8,5} MLD_{50}/ml$ verdünnt. Sterilität und Virustiter werden erneut geprüft.

### 4. Inaktivierung der Viren

Zur Inaktivierung mit Beta-Propiolacton wird das Endvolumen der Virussuspension unter ständigem Rühren bei einer Temperatur von 1 - 4 °C gehalten. Man gibt so viel frisch zubereitete, eisgekühlte wässrige Beta-Propiolacton-Lösung zu, dass eine Konzentration von 1 : 4000 erreicht wird. Nachdem die Suspension 5 min lang bei einer Temperatur von 4 °C gerührt wurde, wird sie in ein zweites Gefäss überführt und weitere 40 Stunden lang gerührt; pH-Wert und Temperatur werden ständig überwacht. Ein Absinken des pH-Wertes ist ein Mass für die BPL-Hydrolyse. Es wird aufgezeichnet. Der pH-Wert fällt von ca. 8,0 auf ca. 7,4. Am Ende der Inaktivierung wird Thiomersal (o-(Ethylquecksilberthio)-benzoesäure) zugesetzt, bis die Konzentration dieser antiseptischen Substanz 1 : 10.000 beträgt.

### 5. Lyophilisieren

Die gemäss Absatz 4 erhaltene inaktivierte Virussuspension wird in Einzeldosen von 1 ml in 3 ml-Fläschchen gefüllt, Lyophilisierstopfen werden lose aufgesetzt und die Vakzine im Vakuum gefriergetrocknet. Wenn der Trocknungsprozess beendet ist, werden die Stopfen festgedrückt und die Fläschchen mit Metallkappen so verschlossen, dass die Stopfen fest in den Fläschchen bleiben. Aufbewahrung der Fläschchen bei einer Temperatur von -20 °C.

### 6. Rekonstituierung zur gebrauchsfertigen Vakzine und Anwendung dieser Vakzine

Zum Gebrauch wird 1 ml steriles destilliertes Wasser durch den Gummistopfen in das Fläschchen injiziert. Dann schüttelt man das Fläschchen vorsichtig, ohne dass sich Schaum bildet, bis die Vakzine vollständig gelöst ist. Der gesamte Inhalt des Fläschchens wird dann subcutan in den Oberarm injiziert.

### 7. Qualitätskontrolle des Endprodukts

Die Qualitätskontrolle umfasst: Bestimmung von Antigenwirkung, Sterilität, Inaktivität, Unschädlichkeit, Stickstoff- und Cholesteringehalt, NaCl, BPL-Rückständen und Thiomersal.

Antigenwirkung: Antigene werden gemäss den Standardanweisungen des National Institute of Health, USA geprüft; ferner werden sie an ihrer Fähigkeit gemessen, im RFFIT-Test Antikörper zu binden. Vergleiche: R.J. Arko et al., Laboratory Techniques in Rabies, 3. Auflage, WHO Monograph Series 23, S. 265 - 267, 1973 und J.S. Smith et al., Lab. Techn. in Rab., 3. Auflage, WHO Monograph Series 23, S. 354 bis 357, 1973.

Sterilität: Alle zur Anwendung kommenden Endprodukte erwiesen sich als steril.

Inaktivität: Diese wird in jedem Fall an 3 jungen Kaninchen und 10 Mäusen getestet, die nach intrazerebraler Inokulation der rekonstituierten Vakzine 14 Tage lang beobachtet werden. Die Tiere zeigten in keinem Fall irgendwelche Krankheitssymptome.

Unschädlichkeit: 3 Meerschweinchen erhalten intraperitoneale Gaben von 5 ml der rekonstituierten Vakzinelösung und 3 Mäuse i.p. Gaben von 0,5 ml.

Die Tiere zeigten in keinem Fall von der Norm abweichende Reaktionen.

Stabilität des nach obiger Beschreibung erhaltenen Impfstoffs in lyophilisierter Form:

Wirksamkeit (AGV-U/ml in % des Ausgangswertes (0 Wert)) nach 3-monatiger Lagerung bei der angegebenen Temperatur.

(a) Stabilität der gemäss Beispiel 1 (Konzentrierung gemäss 2a) gewonnenen Vakzine in lyophilisierter Form.

| Wirksamkeit (AGV-U/ml in % des Ausgangswertes, 0-Wert) | | | |
|---|---|---|---|
| Chargennummer | 0-Wert AGV-U/ml | +37°C 1 Monat | +37°C 2 Monate |
| 83 Ly 111 T16 | 6,7 | 103 % | 110 % |
| 83 Ly 111 T18 | 7,3 | 93 % | 92 % |

(b) Stabilität der gemäss Beispiel 1 (Konzentrierung gemäss 2b) gewonnenen Tollwutvakzine in lyophilisierter Form.

| Wirksamkeit (AGV-U/ml in % des Ausgangswertes, 0-Wert) | | | |
|---|---|---|---|
| Chargennummer | 0-Wert AGV-U/ml | +37°C 1 Monat | +37°C 2 Monate |
| 83 Ly III T15 | 5,0 | 148 % | 98 % |
| 83 Ly III T19 | 8,2 | 107 % | 104 % |
| 83 Ly III T20 | 9,7 | 144 % | 76 % |
| 83 Ly III T21 | 15,3 | 124 % | 92 % |
| 83 Ly III T22 | 8,5 | 165 % | 105 % |
| 83 Ly III T23 | 13,4 | 105 % | 112 % |
| 83 Ly III T24 | 9,5 | 147 % | 126 % |

(c) Wirksamkeit des nach Beispiel 1 gewonnenen lollwutvakzines beim Hund nach s.c. Impfung

| Anzahl geimpfter Tiere mit mehr als | Vakzine gemäss (2a) 7 Hunde | Vakzine gemäss (2b) 8 Hunde |
|---|---|---|
| 0,5 IE | 100 % | 100 % |
| 1 | 86 % | 88 % |
| 2 | 43 % | 75 % |
| IE = Internationale Einheiten des Antikörpergehalts | | |

(d) Wirksamkeit des nach Beispiel 1 gewonnenen Tollwutvakzines beim Menschen

Die Wirksamkeit des neuen Impfstoffs wurde auch mit der der HDC Vakzine (Behring) verglichen. Die nachfolgende Tabelle Zeigt den prozentualen Anteil der Personen, die nach Gabe einer dieser Vakzine mit der Bildung von Antikörpern reagierten, wobei 0,5 IE im allgemeinen als schutzgewährend gelten (Impfung an den Tagen 0, 3, 7, 14, 28).

| Antikörpertiter (RFFIT) geimpfter Pers. am 14. Tag grösser als | Vakzine gem. Beisp. 1 (2b), 3 aufeinanderfolgende Chargen 54 geimpfte Personen | Vakzine gem. Beisp. (2a), 2 aufeinanderfolgende Chargen 15 geimpfte Personen | HDC Vakzine Behring-Werke AGV: 6,3 IE 20 geimpfte Personen |
|---|---|---|---|
| 0,5 IE | 100 % | 100 % | 100 % |
| 2 | 98 % | 100 % | 100 % |
| 5 | 80 % | 80 % | 80 % |
| 10 | 45 % | 60 % | 50 % |
| 15 | 28 % | 47 % | 30 % |
| IE = Internationale Einheiten des Antikörpergehalts | | | |

EP 0 222 974 B1

Ergebnis: Die nach dem erfindungsgemässen Verfahren hergestellte Tollwutvakzine ( = Tollwutimpfstoff) erwies sich im klinischen Versuch einer HDC-Vakzine, d.h. einem Impfstoff, bei dem die Viren in menschlichen Diploidzell (HDC)-Kulturen vermehrt wurden, als mindestens ebenbürtig.

Beispiel 2
Herstellung einer Entenembryo-Vakzine

Analog Beispiel 1 werden Tollwutviren in angebrüteten Enteneiern vermehrt.
Die Viren werden aus den Embryonenköpfen durch Aufschneiden derselben, Entnahme und zellschonende Zerkleinerung des Gehirngewebes freigelegt und durch Suspendieren in einer Phosphatpufferlösung geerntet.
Die Virussuspension wird gemäss Beispiel 1 weiter zum Impfstoff ( = Vakzine) verarbeitet.

Beispiel 3
Entenembryo-Vakzine.

Herstellung der hochkonzentrierten Virussuspension gemäss Beispiel 1. Inaktivierung der Tollwutviren durch Behandlung mit Tri-(n-butyl)-phosphat. Nach der Inaktivierung wird das Konzentrat lyophilisiert.

Biespiel 4
Herstellung von Hühnerembryo-Vakzine

Hühnereier werden 7 Tage lang bei $36\,^{\circ}C \pm 1\,^{\circ}C$ und einer Feuchtigkeit von 60 - 75 % inkubiert. Am 7. Tag der Inkubation wird das Impfvirus direkt in den Dottersack der sich entwickelnden Embryonen in den Eiern inokuliet. Die Inkubation wird fortgesetzt. 10 Tage später werden die Eier geöffnet und die Embryonen herausgenommen. Die Embryonen werden zerlegt. Köpfe, Rückenmark und Rümpfe werden getrennt verarbeitet. Sie werden so zerkleinert, dass sie eine 10 %ige Gewebs-suspension ergeben (d.h. eine Suspension von 10 Gewichtsprozent Embryogewebe). Die Viruskonzentration wird mittels RFFIT-Test (Rapid Fluorescent Focus Inhibition Test) titriert.
Es werden 3 Testreihen durchgeführt; die Ergebnisse sind in der nachfolgenden Tabelle dargestellt.

| | Viruskonzentration angegeben in ID 50/ml |
|---|---|
| Kopf | 4,25 / 4,4 & 4,8 |
| Rückenmark | 3,55 & 4,8 |
| Rumpf | 3,0 / 3,6 & 3,8 |

ID 50/ml = Virustiter x $\log_{10}$ für die Mindestkonzentration zur Infizierung von 50 96 der Gewebekulturen. Es wurde festgestellt, dass das Gewebe des zentralen Nervensystems (ZNS) ca. 10mal so viel Virus enthielt wie der Rumpf ohne ZNS.
Aufgrund dieses Ergebnisses erfolgte die Herstellung der Hühnerembryo-Vakzine nach der im Beispiel I beschriebenen Methode allein aus den Embryonenköpfen.
Im vorliegenden Fall wird das an Hühnerzellen angepasste Tollwutvirus in teilweise inkubierten Hühnereiern vermehrt, z.B. nach der Methode von H. Koprowsky, Laboratory Technique in Rabies by M.M.
Kaplan et al., WHO Genf, Kapitel 26, S. 235 - 242. Am 7. Tag der Inkubation werden die Eier inokuliert; am folgenden Tag wird weiter inkubiert. Neun bis zehn Tage nach Inokulation des Virus in den Dottersack werden die Köpfe der Hühnerembryonen entnommen und gemäss der in Beispiel 1 beschriebenen Methode verarbeitet. Dabei erfolgt die Endkonzentrierung der Viren durch Fällung mit Polyethylenglykol. Die derart hergestellte Vakzine wird den unter 1.7 geschilderten Qualitätskontrollen unterzogen. Auch dieser Impfstoff erwies sich beim Menschen als voll wirksam.

Beispiel 5
Herstellung von Wachtelembryo-Vakzine

Analog wie im Biespiel 1 beschrieben, werden attenuierte Tollwutviren in angebrüteten Wachteleiern vermehrt, geerntet und zum Impfstoff aufgearbeitet.
Erhaltener Impfstoff erwies sich im Tierversuch als voll wirksam.

Beispiel 6
Herstellung einer Tollwutvakzine aus in Mäusen vermehrten Viren.

An Mäuse attenuierte Tollwutsaatviren werden 5 Tage alten Mäusen intracerebral inokuliert. Nach 10 Tagen werden die noch überlebenden Mäuse getötet. Die Gehirne der Tiere werden entnommen und zellschonend zerkleinert. Die Zellsuspension wird nach dem Verfahren des Beispiel 1 zum Impfstoff verarbeitet.

Beispiel 7
Herstellung einer Tollwutvakzine aus in Ratten vermehrten Viren.

An Ratten attenuierte Tollwutsaatviren werden 3 - 4 Tage alten Ratten intracerebral inokuliert.
Nach 12 Tagen werden die noch überlebenden Ratten getötet. Die Gehirne der Tiere werden analog Beispiel 6 zum Impfstoff verarbeitet.

Beispiel 8
Tollwutvakzine aus in Kaninchen vermehrten Viren.

An Kaninchen attenuierte Saatviren werden 6 Tage alten Kaninchen intracerebral inokuliert. Nach 15 Tagen werden die Kaninchen getötet. Die Gehirne der Tiere werden analog Beispiel 6 zum Impfstoff verarbeitet.

Beispiel 9
Vakzine aus in Lämmern vermehrten Viren.

An Schafe attenuierte Tollwutsaatviren werden 8 - 10 Tage alten Lämmern intracerebral inokuliert.
Nach 30 Tagen werden die Lämmer geopfert. Ihre Gehirne werden entnommen und analog Beispiel 6 zum Impfstoff verarbeitet.

**Patentansprüche**

1.  Verfahren zur Herstellung eines Tollwutimpfstoffes durch Vermehrung von Tollwutviren in tierischem Nervengewebe, vorwiegend in Geflügelembryonen, Ernte der Viren aus dem Nervengewebe, Anreicherung der Viren, Inaktivierung derselben und Konfektionierung zum Impfstoff, dadurch gekennzeichnet, dass man bei der Ernte der Viren die Homogenisierung des Nervengewebes mit einem Mixer vermeidet und dadurch verhindert, dass unvollständige Virionen extrahiert werden, indem man das Nervengewebe zellschonend zerkleinert, aus der erhaltenen Zellsuspension die vollständigen, lebenden und vermehrungsfähigen Virionen herausspült, die erhaltene Virionensuspension reinigt, durch Extraktion mit einem mit Wasser nicht mischbaren organischen Lösungsmittel von Lipiden befreit und danach selektiv konzentriert.

2.  Verfahren nach Patentanspruch 1, dadurch gekennzeichnet, dass man die Viren in Geflügelembryonen vermehrt und danach die virushaltigen Embryonenköpfe mit einem Fleischwolf zerkleinert oder zerschneidet oder dass man nach Öffnung der Köpfe das Hirngewebe entnimmt und danach zellschonend zerkleinert.

3.  Verfahren nach Patentanspruch 1, dadurch gekennzeichnet, dass man die Viren aus dem zerkleinerten Gewebe mit einer Pufferlösung, vorzugsweise mit einer wässrigen Pufferlösung vom pH 7 bis 8 extrahiert.

4.  Verfahren nach Patentanspruch 1, dadurch gekennzeichnet, dass man für die Extraktion der Lipide als Lösungsmittel flüssige, leicht flüchtige, gegebenenfalls halogenierte Kohlwasserstoffe verwendet.

5.  Verfahren gemäss Patentanspruch 4, dadurch gekennzeichnet, dass man als Lösungsmittel Petrolether, fluoriertes und chloriertes Ethan oder nahe Homologen derselben, verwendet.

6.  Verfahren gemäss Patentanspruch 5, dadurch gekennzeichnet, dass man als Lösungsmittel Heptane verwendet.

7. Verfahren nach Patentanspruch 1, dadurch gekennzeichnet, dass man die von Lipiden weitgehend befreite Virussuspension durch Dichtegradientenzentrifugation und Fällung mit einem Polyethylenglykol, vorzugsweise mit PEG 6000, weiter konzentriert.

8. Verfahren nach Anspruch 1, dadurch gekennzeichnet, dass für die Vermehrung der Tollwutviren angebrütete Eier von Enten, Hühnern oder Wachteln verwendet werden.

## Claims

1. Process for the preparation of a rabies vaccine by replication of rabies viruses in animal nerve tissue, predominantly in poultry embryos, harvesting of the viruses from the nerve tissue, concentration of the viruses, inactivation thereof and formulation to give a vaccine, characterised in that homogenisation of the nerve tissue using a mixer is avoided and as a result extraction of incomplete virions is prevented during harvesting by comminuting the nerve tissue without damaging the cells, rinsing out the complete virions, which are viable and capable of replication, from the cell suspension obtained, purifying the virion suspension obtained, freeing it from lipids by extraction with a water-immiscible organic solvent and then selectively concentrating it.

2. Process according to Patent Claim 1, characterised in that the viruses are replicated in poultry embryos and the virus-containing embryonic heads are then comminuted or cut up using a meat mincer or in that after opening the heads the cerebral tissue is taken out and then comminuted without damaging the cells.

3. Process according to Patent Claim 1, characterised in that the viruses are extracted from the comminuted tissue using a buffer solution, preferably using an aqueous buffer solution of pH 7 to 8.

4. Process according to Patent Claim 1, characterised in that liquid, easily volatile, optionally halogenated hydrocarbons are used as solvents for the extraction of the lipids.

5. Process according to Patent Claim 4, characterised in that petroleum ether, fluorinated and chlorinated ethane or close homologues thereof are used as solvents.

6. Process according to Patent Claim 5, characterised in that heptanes are used as solvents.

7. Process according to Patent Claim 1, characterised in that the largely lipid-free virus suspension is further concentrated by density gradient centrifugation and precipitation with a polyethylene glycol, preferably with PEG 6000.

8. Process according to Claim 1, characterised in that partially incubated ducks', hens' or quails' eggs are used for the replication.

## Revendications

1. Procédé de préparation d'un vaccin antirabique par multiplication de virus de la rage dans les tissus nerveux animaux, surtout dans des embryons de volaille, récolte des virus à partir du tissu nerveux, enrichissement des virus, inactivation de ces virus et transformation en un vaccin,
caractérisé en ce que dans la récolte des virus, on évite l'homogénéisation du tissu nerveux avec un mélangeur et on empêche l'extraction de virions incomplets, en broyant le tissu nerveux de manière à épargner les cellules, en lavant à partir de la suspension cellulaire obtenue les virions entiers, vivants, et réplicables, en purifiant la suspension de virions obtenue, en la débarassant des lipides par extraction avec un solvant organique non miscible à l'eau et en concentrant de façon sélective.

2. Procédé selon la revendication 1, caractérisé en ce qu'on multiplie le virus dans des embryons de poulet, puis qu'on broie ou dissèque les têtes d'embryons contenant le virus avec un hâchoir ou en ce qu'après ouverture de la tête on prélève le tissu cérébral puis en ce qu'on broie en épargnant les cellules.

3. Procédé selon la revendication 1, caractérisé en ce qu'on extrait les virus à partir du tissu broyé avec une solution tampon, de préférence avec une solution tampon aqueuse à pH 7 à 8.

4. Procédé selon la revendication 1, caractérisé en ce qu'on utilise pour l'extraction des lipides, comme solvants, des hydrocarbures liquides, légèrement volatils, éventuellement halogénés.

5. Procédé selon la revendication 4, caractérisé en ce qu'on utilise comme solvants, de l'éther de pétrole, de l'éthane fluoré et chloré ou des homologues proches de ces corps.

6. Procédé selon la revendication 5, caractérisé en ce qu'on utilise comme solvant l'heptane.

7. Procédé selon la revendication 1, caractérisé en ce qu'on concentre plus avant la suspension virale largement débarassée des lipides par centrifugation à gradient de densité et précipitation avec un polyéthylène glycol, de préférence avec du PEG 6000.

8. Procédé selon la revendication 1, caractérisé en ce que pour la multiplication des virus de la rage, on utilise des oeufs couvés de canard, de poule ou de caille.